# EUROPÄISCHE PATENTANMELDUNG

(11) **EP 3 088 383 A1**
(43) Veröffentlichungstag der Anmeldung: **02.11.2016**
(21) Anmeldenummer: 15165743.4
(22) Anmeldetag: 29.04.2015
(51) Int. Cl.: C07C 205/03, C07C 205/51, C07C 227/04

(54) **OPTIMIERTE SYNTHESE VON PREGABALIN SOWIE 4-AMINOBUTANSÄUREN UNTER EINSATZ EINES VERBESSERTEN HERSTELLVERFAHRENS VON KONJUGIERTEN NITROALKENEN**

(71) Anmelder: K.H.S. Pharma Holding GmbH, 55218 Ingelheim am Rhein (DE)
(72) Erfinder: STUMPF, MARCUS, 55268 Nieder-Olm (DE)
(74) Vertreter: Drescher, Christian

(57) **Zusammenfassung**

Die vorliegende Erfindung beschreibt eine optimierte Synthese von Pregabalin sowie weiteren 4-Aminobutansäuren unter Einsatz eines verbesserten Herstellverfahrens von konjugierten Nitroalkenen. Dies wird erreicht durch direkt aufeinanderfolgende Nitro-Aldol-Reaktion eines aliphatischen Aldehyds mit einem Nitroalkan in Gegenwart eines geeigneten Diamins und Eliminierung des konjugierten Nitroalkens durch Zugabe einer Säure, anschließender katalysierter asymmetrischer 1,4-Michael-Addition eines geeigneten Nucleophils, einer daran anschließenden Retro-Claisen-Reaktion bzw. einer Verseifung und Decarboxylierung mit abschließender Reduktion zur 4-Aminobutansäure.

## Beschreibung

### EINLEITUNG

Pregabalin ist ein Arzneistoff aus der Gruppe der Antikonvulsiva. Medikamente enthaltend Pregabalin sind zugelassen für die Behandlung von neuropathischen Schmerzen, Epilepsie und generalisierten Angststörungen.

Pregabalin wird unter anderem unter dem Namen LYRICA^{®} vermarktet.

Chemisch gesehen ist Pregabalin (S)-3-(Aminomethyl)-5-methylhexansäure und weist folgende Strukturformel auf:

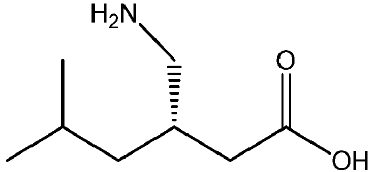

Es ist eine Vielzahl von recht unterschiedlichen Verfahren zur Synthese von Pregabalin bekannt, beispielsweise mit einer enantioselektiven Hydrierung oder einer Racematspaltung als Schlüsselschritt. Die meisten dieser Synthesen sind dabei für die großtechnische Produktion von Pregabalin, welches jährlich im 100 Tonnen Maßstab hergestellt wird, nicht geeignet.

Die vorliegende Erfindung beschreibt eine optimierte Synthese von Pregabalin sowie weiteren 4-Aminobutansäuren unter Einsatz eines verbesserten Herstellverfahrens von konjugierten Nitroalkenen.

Dies wird erreicht durch direkt aufeinanderfolgende Nitro-Aldol Reaktion eines aliphatischen Aldehyds mit einem Nitroalkan in Gegenwart eines geeigneten Diamins und Eliminierung zum konjugierten Nitroalken durch Zugabe einer Säure, anschließender katalysierter asymmetrischer 1,4-Michael-Addition eines geeigneten Nucleophils, einer daran anschließenden Retro-Claisen-Reaktion beziehungsweise einer Verseifung und Decarboxylierung mit abschließender Reduktion zur 4-Aminobutansäure.

Im ersten Reaktionsschritt wird ein aliphatischer Aldehyd (1) in Gegenwart eines Diamins (2) mit einem Nitroalkan (3) versetzt und anschließend mit einer Säure (4) gemäß folgendem Reaktionsschema zu einem 1-Nitroalk-1-en (5) umgesetzt.

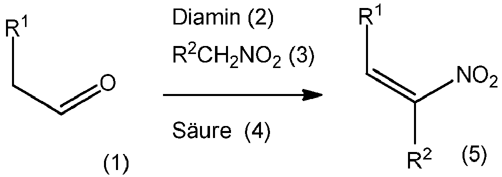

Das Diamin (2) fungiert bei dieser Nitro-Aldol-Reaktion als Katalysator, der anschließend wiedergewonnen werden kann. Geeignete Diamine (2) weisen sowohl eine primäre Aminfunktion als auch eine tertiäre Aminstruktur auf, die durch eine Brücke mit 2 oder 3 Kohlenstoffatomen verbunden sind. Das primäre Amin des Diamins aktiviert dabei zunächst den Aldehyd durch Bildung eines Imins, wogegen das tertiäre Amin das Nitromethan deprotoniert. Aus dem so gebildeten Intermediat wird das 1-Nitroalk-1-en (5) durch Eliminierung gebildet. Diese Eliminierung findet gemäß literaturbeschriebener Methoden unter basischen Bedingungen statt und wird durch Energiezufuhr, d.h. bei erhöhter Temperatur, erzwungen. Dies ist bei aromatischen Aldehyden wie denen in Catalysis Letters 57 (1999) 227-231 beschriebenen unproblematisch, da diese einerseits 1-Nitroalk-1-ene sehr leicht eliminieren und andererseits keine Isomerisierung des 1-Nitroalk-1-ens zur entsprechenden allylischen Nitroverbindung stattfinden kann. Wie in J. Org. Chem. 1986, 51, 4368-4375 aufgezeigt wird, isomerisieren aliphatische 1-Nitroalk-1-ene unter literaturbekannten Reaktionsbedingungen sehr leicht zu den entsprechenden allylischen Nitroverbindungen, welche daher in der Regel als Hauptprodukt erhalten werden, während das 1-Nitroalk-1-en nur in geringen Ausbeuten von unter 50% entsteht.

Die Erfinder der vorliegenden Anmeldung haben nun gefunden, dass die Eliminierung des 1-Nitroalk-1-ens (5) aus dem zwischenzeitlich gebildeten Imin durch Zugabe von Säure sehr effizient und unter milden Bedingungen erreicht werden kann, ohne dass es zu Nebenreaktionen, insbesondere zur Bildung von allylischen Nitroverbindungen, kommt.

Die Eliminierung kann im Rahmen der Aufarbeitung erfolgen, wobei sich das Diamin (2) in der wässrigen Phase löst, aus der es mittels Flüssig-Flüssig-Extraktion wiedergewonnen werden kann. Das 1-Nitroalk-1-en (5) hingegen verbleibt in der organischen Phase in Lösung und kann ohne Aufreinigung im nächsten Schritt eingesetzt werden. Die Lösung ist auch bei -20 °C lagerbar.

Für die vorliegende Erfindung geeignete aliphatische Aldehyde (1) sind solche mit verzweigtem oder unverzweigtem Alkylrest der allgemeinen Formel R¹CH₂CHO. Besonders geeignete Aldehyde sind solche mit R¹ = C*ₙ*H_{2*n*+1} mit *n* = 0 bis 8. Spezielle Beispiele für geeignete Aldehyde sind Ethanal (Acetaldehyd), Propanal (Propionaldehyd), n-Butanal (Butyraldehyd), n-Pentanal (Valeraldehyd), 3-Methylbutanal (Isovaleraldehyd) und n-Hexanal (Capronaldehyd), bevorzugt ist 3-Methylbutanal (Isovaleraldehyd).

Für die vorliegende Erfindung geeignete Diamine (2) weisen sowohl eine primäre Aminfunktion als auch eine tertiäre Aminstruktur auf, die durch eine Brücke mit 2 bis 3 Kohlenstoffatomen verbunden sind. Die Alkylreste an der tertiären Aminfunktion können jeweils unabhängig voneinander Methyl-, Ethyl- oder Propylgruppen sein, alternativ funktioniert auch ein cyclisches Amin. Beispiele für geeignete Diamine sind daher N,N-Dimethylethan-1,2-diamin, N,N-Diethylethan-1,2-diamin, N,N-Dipropylethan-1,2-diamin, N,N-Dimethylpropan-1,3-diamin, N,N-Diethylpropan-1,3-diamin, N,N-Dipropylpropan-1,3-diamin, 2-Aminoethylpyrrolidin, 3-Aminopropylpyrrolidin, 2-Aminoethylpiperidin oder 3-Aminopropylpiperidin. Bevorzugtes Diamin ist N,N-Dimethyl-1,2-ethandiamin.

Für die vorliegende Erfindung geeignete Nitroalkane (3) weisen einen verzweigten oder unverzweigten Alkylrest auf und haben die allgemeine Formel R²CH₂NO₂, wobei R² = C*ₘ*H_{2*m*+1} mit m = 0 bis 4 sein kann. Beispiele für geeignete Nitroalkane sind Nitromethan, Nitroethan, 1-Nitropropan, 1-Nitrobutan sowie 1-Nitropentan. Ganz besonders bevorzugtes Nitroalkan ist Nitromethan.

Bei der Verwendung von Nitroalkanen (3) mit m > 0 entstehen 1-substituierte 1-Nitroalk-1-ene. Diese können alternativ auch durch Reaktion von entsprechenden aliphatischen Ketonen mit Nitromethan erhalten werden. Schließlich sind 1-disubstituierte 1-Nitroalk-1-ene über die Umsetzung von aliphatischen Ketonen mit Nitroalkanen (3) mit m > 0 zugänglich.

Für die vorliegende Erfindung geeignete Säuren (4) sind Carbonsäuren sowie verdünnte Mineralsäuren mit einem pKa-Wert von unter 5. Beispiele für geeignete Säuren sind daher Ameisensäure, Essigsäure (AcOH), Propionsäure, Benzoesäure, Salzsäure, Schwefelsäure sowie Salpetersäure.

Im Fall der Umsetzung von Isovaleraldehyd mit Nitromethan in Gegenwart von N,N-Dimethylethan-1,2-diamin und Eliminierung von (E)-4-Methyl-1-nitropent-1-en durch Zugabe von Essigsäure wird das 4-Methyl-1-nitropent-1-en als Zwischenstufe der Synthese von Pregabalin mit einer Ausbeute von 88 % erhalten, wobei zu mehr als 95 % die (E)-Konfiguration entsteht.

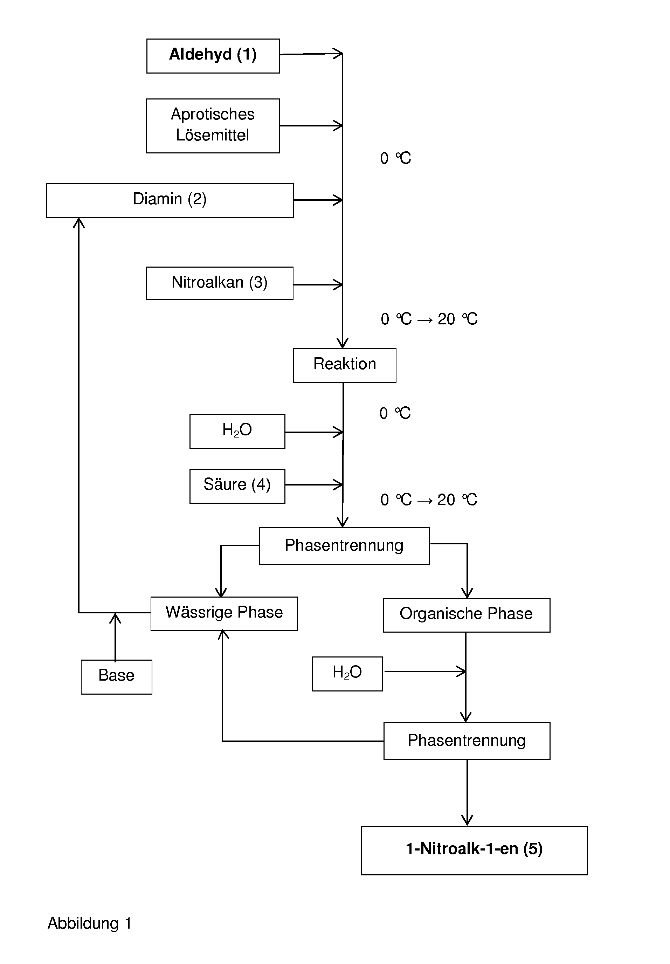

Ein detailliertes Fließschema der großtechnischen Synthese von 1-Nitroalk-1-enen (5) ist in Abbildung 1 wiedergegeben.

Im zweiten Reaktionsschritt wird das 1-Nitroalk-1-en (5) in Gegenwart eines Katalysators (6) mit einem Nucleophil (7) in einer asymmetrischen 1,4-Michael-Addition zu einem Nitroester der allgemeinen Formel (8) umgesetzt.

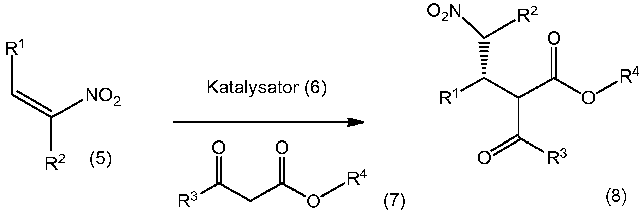

R¹ und R² sind dabei wie oben definiert.

Zunächst wird dabei der Katalysator (6) zu einer Lösung des Nitroalkens (3) gegeben und das resultierende Gemisch auf -20 °C gekühlt. Bei dieser Temperatur wird das Nucleophil (7) zugegeben und bis zum vollständigen Umsatz gerührt. Die Lösung enthält dann den Nitroester (8) in hoher Ausbeute mit einem ebenfalls hohen Überschuss des gewünschten Enantiomers.

Für die vorliegende Erfindung geeignete Nucleophile (7) sind Acetessigester und Dialkylmalonate der allgemeinen Formel R³COCH₂COOR⁴ mit R³ = C*ₚ*H_{2*p*+1} oder OC*ₚ*H_{2*p*+1} mit *p* = 1 bis 3 sowie R⁴ = C*_{q}*H_{2*q*+1} mit q = 1 bis 3 sowie 2,2-Dimethyl-1,3-dioxan-4,6-dion (Meldrumsäure). Beispiele für erfindungsgemäße Nucleophile sind 3-oxo-butansäuremethylester; 3-oxo-butansäureethylester (Ethylacetoacetat), 3-oxo-butansäurepropylester, 3-oxo-butansäureisopropylester, Malonsäuredimethylester, Malonsäurediethylester, Malonsäuredipropylester, Malonsäuredüsopropylester. Bevorzugte Nucleophile sind Ethylacetoacetat sowie Malonsäurediethylester.

Für die Durchführung der erfindungsgemäßen Reaktion eignen sich als Katalysatoren (6) besonders Chininderivate, deren Herstellung beispielsweise aus US 7,582,764 B2 oder Liu et al, Tetrahedron 67 (2011) 636-640 bekannt ist. Als besonders geeignete Katalysatoren (6) für die asymmetrische 1,4-Michael-Addition haben sich Chinidin und dessen Derivate erwiesen. Beispielsweise katalysiert Cupreidin (Desmethylchinidin) die Umsetzung von (E)-4-Methyl-1-nitropent-1-en mit Acetessigester zu (3S)-Ethyl-2-acetyl-5-methyl-3-(nitromethyl)hexanoat mit einer Selektivität der Hydrierung von 96:4 zugunsten des gewünschten (3S)-Enantiomers. Bevorzugter Katalysator ist daher Cupreidin.

Die Katalysatoren lösen sich vollständig in der Reaktionslösung, wird jedoch aus dieser leicht durch mehrfaches Extrahieren der organische Phase und fraktionierter Kristallisation praktisch quantitativ zurückgewonnen und können so erneut eingesetzt werden.

Für die Durchführung des ersten Reaktionsschrittes sowie des zweiten Reaktionsschrittes geeignete Lösemittelsysteme kommen prinzipiell alle aprotischen, nicht mit Wasser mischbaren Lösemittel in Frage. Großtechnisch bevorzugt sind hingegen Lösemittel, welche eine azeotrope Destillation mit/von Wasser ermöglichen. Beispiele für erfindungsgemäße Lösemittel sind daher Toluol, Benzol, Dichlormethan, Chloroform, Tetrachlorkohlenstoff, Tetrahydrofuran (THF), Dimethylformamid (DMF), Dimethylsulfoxid (DMSO) sowie Pentan, Hexan, oder Heptan.

Bei der Synthese von Pregabalin wird (E)-4-Methyl-1-nitropent-1-en mit Acetessigester in Gegenwart katalytischer Mengen von Cupreidin umgesetzt, wobei das Reaktionsprodukt Ethyl-2-acetyl-5-methyl-3-(nitromethyl)hexanoat in einer Ausbeute von 97 % mit einem Überschuss des (3S)-Enantiomers von 93 % erhalten wird. Das Reaktionsprodukt muss wiederum nicht aufgearbeitet werden, sondern die Lösung kann direkt für den nächsten Schritt verwendet werden.

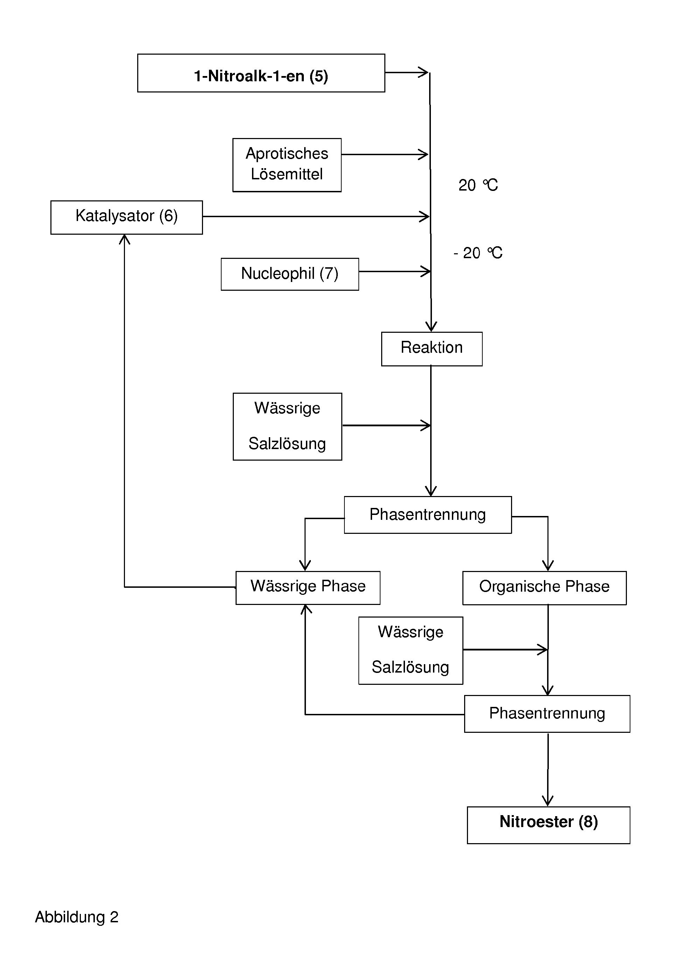

Ein detailliertes Fließschema der großtechnischen Synthese von Nitroestern (8) ist in Abbildung 2 wiedergegeben.

Im dritten und vierten Reaktionsschritt kann der Nitroester (8) auf verschiedene Art und Weise zu 4-Aminobutansäuren der Formel (12) umgesetzt werden.

Aus Dialkylmalonaten dargestellte Nitroester (8) können durch Methoden wie aus Tetrahedron 67 (2011), 636-640 bekannt zunächst hydriert und anschließend decarboxyliert werden. Diese Reaktionsführung eignet sich allerdings nicht für die Weiterverarbeitung von aus Acetessigestern dargestellten Nitroestern (8), da das bei der Hydrierung entstehende Amin intramolekular mit der Ketofunktion unter Ausbildung eines Imins reagiert. Dieses Imin wird durch das Hydrierungsmittel leicht weiter zu einem Pyrrolidin reduziert wird und führt daher nicht zum gewünschten Produkt.

Sowohl aus Acetessigestern als auch aus Dialkylmalonaten hergestellte Nitroester (8) können jedoch in einer Kombination von Retro-Claisen Reaktion bzw. Verseifung / Decarboxylierung und anschließender Hydrierung in die entsprechenden, gegebenenfalls 3- und/oder 4-alkylsubstituierten 4-Aminobutansäuren (12) überführt werden, weshalb dieser Prozess bevorzugt ist.

Bei der erfindungsgemäßen Reaktionsfolge wird der Nitroester (8) zunächst durch den Zusatz einer Base (9) zu einem Salz der entsprechenden Nitrosäure (10) carboxyliert, welche ohne zwischenzeitliche Aufarbeitung anschließend mittels eines Hydrierungsmittels (11) direkt zur Aminobutansäure (12) reduziert wird.

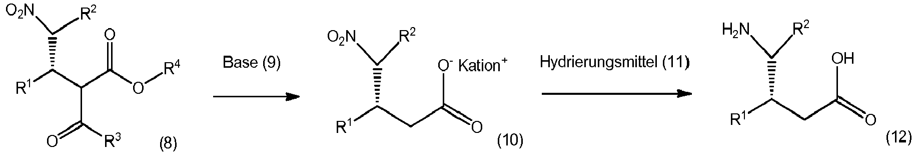

R¹, R², R³ und R⁴ sind dabei wie oben definiert.

Diese Verfahrensführung ist auch deshalb vorteilhaft, weil beide Reaktionsschritte im gleichen Lösemittelsystem erfolgen können und dadurch ein zeit- und kostenaufwendiger Aufarbeitungsschritt eingespart werden kann.

Für die Durchführung des erfindungsgemäßen Verfahrens geeignete Basen (9) sind alle starken Basen, beispielsweise Alkali- oder Erdalkalihydroxide wie Kaliumhydroxid (KOH), Natriumhydroxid (NaOH), Magnesiumhydroxid oder Calciumhydroxid. Die bevorzuge Base ist KOH.

Für die Durchführung des erfindungsgemäßen Verfahrens eignen sich übliche Hydrierungsmittel (11) wie beispielsweise Raney-Nickel, Wasserstoff/ Palladiumkatalysator, Wasserstoff/Platinkatalysator oder Wasserstoff/Iridiumkatalysator. Das bevorzugte Hydrierungsmittel ist Raney-Nickel.

Für die Durchführung der Reaktion geeignete Lösemittelsysteme sind alle protischen Alkohol/Wasser Systeme wie Methanol/Wasser oder Ethanol/Wasser. Wegen ihrer Eignung in großtechnischen Verfahren sind Gemische von Methanol und Wasser besonders bevorzugt.

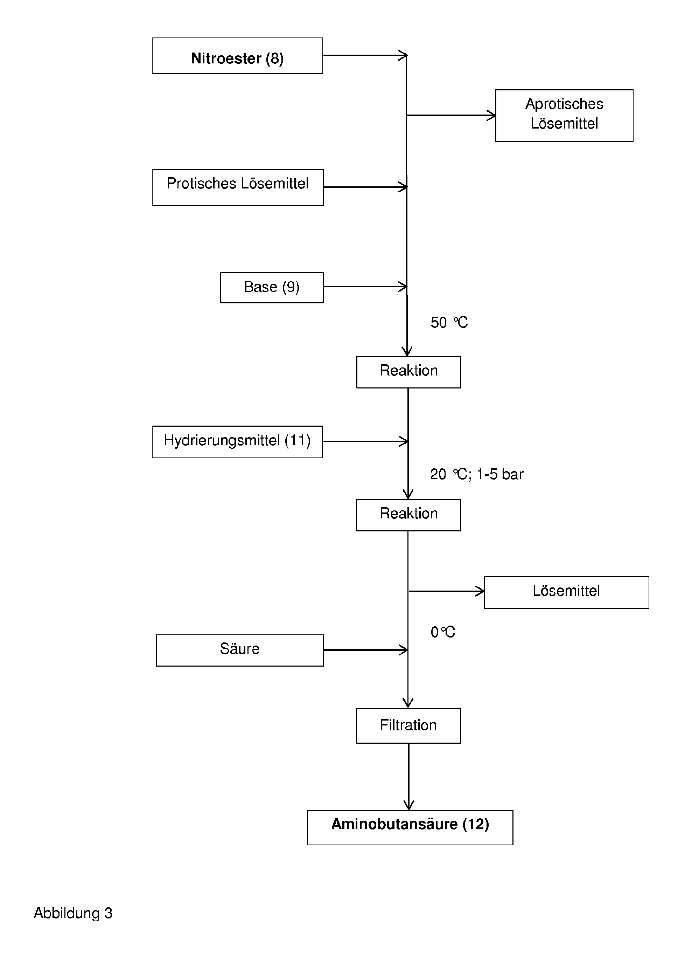

Ein detailliertes Fließschema der erfindungsgemäßen großtechnischen Synthese von Aminobutansäuren (12) ist in Abbildung 3 wiedergegeben.

Eine besonders bevorzugte Ausführungsform der vorliegenden Erfindung ist ein Eintopfverfahren zur Herstellung von Pregabalin umfassend folgende Schritte:
(a) Umsetzung von Isovaleraldehyd mit Nitromethan in Gegenwart eines Diamins der Formel (2) und Eliminierung von (E)-4-Methyl-1-nitropent-1-en durch Zugabe einer Säure (4);
(b) Umsetzung von (E)-4-Methyl-1-nitropent-1-en mit Acetessigester in Gegenwart katalytischer Mengen von Cupreidin zu (3S)-Ethyl-2-acetyl-5-methyl-3-(nitromethyl)hexanoat;
(c) Umsetzung von (3S)-Ethyl-2-acetyl-5-methyl-3-(nitromethyl)hexanoat mit einer Base (9) zu Kalium-(S)-5-methyl-3-(nitromethyl)hexanoat; und
(d) Umsetzung von Kalium-(S)-5-methyl-3-(nitromethyl)hexanoat mit einem Hydrierungsmittel zu Pregabalin und abschließende Kristallisation von Pregabalin durch Zugabe einer zweiten Säure.

Bevorzugtes Diamin der Formel (2) ist dabei N,N-Dimethylethan-1,2-diamin, bevorzugte Säure (4) sowie bevorzugte zweite Säure ist Essigsäure, bevorzugte Base (9) ist Kaliumhydroxid und bevorzugtes Hydrierungsmittel ist Raney-Nickel.

Die Eintopfsynthese von Pregabalin verläuft daher bevorzugt gemäß folgendem Schema:

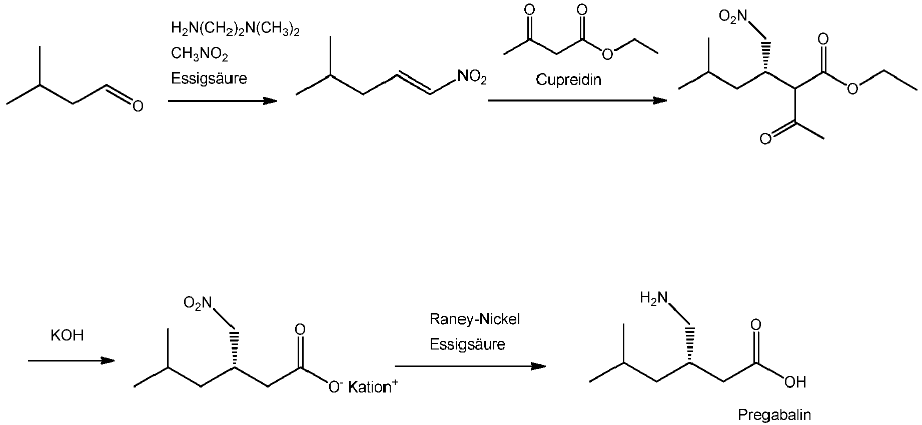

Insgesamt stellt die vorliegende Erfindung ein verbessertes Verfahren zur Herstellung einer Vielzahl von synthetisch und/oder medizinisch wertvollen Verbindungen zur Verfügung, insbesondere optional am C₃-Atom und/oder am C₄-Atom alkylsubstituierten 4-Aminobutansäuren der Formel (12). Bevorzugte alkylsubstituierte 4-Aminobutansäure ist die (S)-3-(Aminomethyl)-5-methylhexansäure (Pregabalin).

Des weiteren stellt die vorliegende Erfindung neue Zwischenprodukte für die Pragabalin-Synthese zur Verfügung, nämlich (3S)-Ethyl-2-acetyl-5-methyl-3-(nitromethyl)hexanoat sowie Salze der (S)-5-methyl-3-(nitromethyl)hexansäure, bevorzugt Kalium-(S)-5-methyl-3-(nitromethyl)hexanoat.

### BEISPIELE

### Beispiel 1: Darstellung von (E)-4-Methyl-1-nitropent-1-en

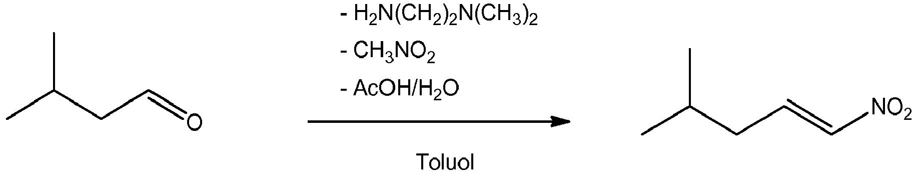

15,0 g Isovaleraldehyd (97 %ig, 0,16 mol) und 15,19 g N,N-Dimethyl-1,2-ethandiamin (98 %ig, 0,16 mol) werden mit 35 mL Toluol vermischt und bei 0 bis 3 °C innerhalb von 10 min 12,31 g Nitromethan (98 %ig, 0,19 mol) zugegeben. Die Kühlung wird abgestellt und die trübe Lösung für drei Stunden gerührt, wobei sie sich auf 20-25 °C erwärmt. Nach 155 Minuten wird die Lösung wieder auf 0°C gekühlt und 35 mL Wasser sowie 19,5 mL Essigsäure zugegeben. Die milchige Lösung wird für zwei Stunden gerührt, anschließend die Phasen getrennt und die organische Phase mit 35 mL Wasser extrahiert. Nach der Phasentrennung wird die organische Phase mittels azeotroper Destillation getrocknet und ohne weitere Aufreinigung für die nächste Stufe verwendet. Die erhaltene Lösung (121,2 g) enthält 19,39 g (E)-4-Methyl-1-nitropent-1-en (mittels NMR bestimmt), was einer Ausbeute von 89 % entspricht.

¹H NMR (500 MHz, CDCl₃) δ = 1.10 (d, *J*=6.62 Hz, 7 H) 1.85 - 2.00 (m, *J*=13.44, 6.65, 6.65, 6.65, 6.65 Hz, 1 H) 2.22 (ddd, *J*=8.12, 6.70, 1.26 Hz, 1 H) 7.05 (d, *J*=13.32, 1 H) 7. 19 (dt, *J*=13.32, 7.88 Hz, 1 H) ppm. Nur die (E)-Konfiguration ist sichtbar.

¹³C NMR (CDCl₃) δ = 21.3, 22.1, 27.7, 37.1, 55.2, 76.7, 77.2, 92.8, 125.2, 128.1, 128.9, 137.7, 141.3, 161.5 ppm.

### Beispiel 1.1: Wiedergewinnung des Katalysators N,N-Dimethylethan-1,2-diamin

Während das (E)-4-Methyl-1-nitropent-1-en in der organischen Phase verbleibt, löst sich das Dimethylethylendiamin in der wässrigen Phase, aus der es mittels Flüssig-Flüssig-Extraktion mit Toluol bei pH 14 mit einer Ausbeute von 77 % wiedergewonnen wird.

### Beispiel 2a: Darstellung von (3S)-Ethyl-2-acetyl-5-methyl-3-(nitromethyl)hexanoat

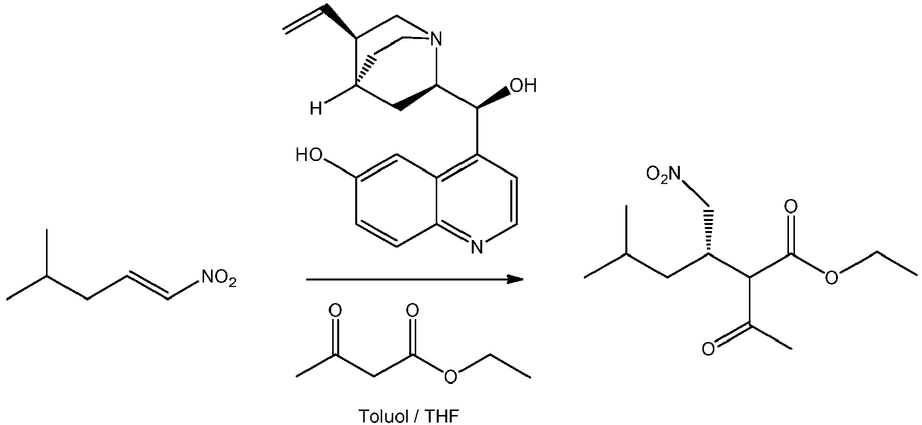

Zu 17,78 g (137,63 mmol) (E)-4-Methyl-1-nitropent-1-en in 70ml Toluol und 30 mL wasserfreiem THF werden 4,27 g Cupreidin (13,76 mmol) gegeben, die Lösung auf -20 °C gekühlt und 35,82 g Ethylacetoacetat (275,26 mmol) zugegeben. 24 Stunden wird bei dieser Temperatur gerührt. Anschließend wird das Reaktionsgemisch zwei Mal mit je 50 mL 1 N HCl und anschließend mit 50 mL gesättigter, wässriger NH₄Cl-Lösung extrahiert. Nach der Phasentrennung wird die organische Phase mittels azeotroper Destillation getrocknet, abfiltriert und der Gehalt mittels NMR über Trimethoxybenzol als interner Standard bestimmt. Man erhält eine 25 %-ige Lösung von (3S)-Ethyl-2-acetyl-5-methyl-3-(nitromethyl)hexanoat in Toluol mit einem Gesamtgewicht von 144 g (entsprechend einer Ausbeute von 97 %, Enantiomerenüberschuss 93 %). Die Ausbeutebestimmung erfolgt mittels HPLC (vs. mittels Säulenchromatographie (Ethylacetat / Cyclohexan 1 / 10) gereinigtem Standard) und NMR mit Trimethoxybenzol als internem Standard.

¹H NMR (CDCl₃, 500MHz): δ = 4.53 - 4.69 (m, 2 H), 4.21 - 4.28 (m, 2 H), 3.79 (d, *J*=6.3 Hz, 1 H) bzw. 3.77 (d, *J*=6.3 Hz, 1 H), 2.89 - 2.33 (m, 1 H), 2.36 (s, 3 H) bzw. 2.33 (s, 3 H), 1.60 - 1.74 (m, 1 H), 1.35 - 1.39 (m, 1 H der diastereotope CH₂), 1.34 (td, *J*=7.1, 1.3 Hz, 3 H), 1.18 - 1.24 (m, 1 H der diastereotope CH₂), 0.80 - 1.04 (m, 6 H) ppm.

¹³C NMR (CDCl₃, 126MHz): δ = 202.1, 201.7, 167.3, 167.5, 76.9, 77.3, 62.4, 62.2, 60.9, 61.0, 39.4, 38.4, 34.6, 34.6, 30.6, 30.1, 25.6, 25.5, 23.1, 22.4, 22.0, 21.5, 14.1 ppm.

Der Enantiomerenüberschuss wird mittels Chromatographie auf chiraler stationärer Phase (Chiracel OD-H, Laufmittel *n*-Heptan/2-Propanol 92:8) bestimmt.

### Beispiel 2.1: Wiedergewinnung des Katalysators Cupreidin

Die beiden wässrigen Phasen aus den Extraktionen bei 2a) werden vereinigt und der pH mit NaOH auf 6 gestellt. Dabei kristallisiert das Cupreidin aus. Die Suspension wird auf 0 °C abgekühlt und eine Stunde nachgerührt. Mit dieser Methode können 4,24 g des Katalysators zurück gewonnen werden (99 % Ausbeute).

### Beispiel 2b: Darstellung von (3S)-Ethyl-2-acetyl-5-methyl-3-(nitromethyl)hexanoat

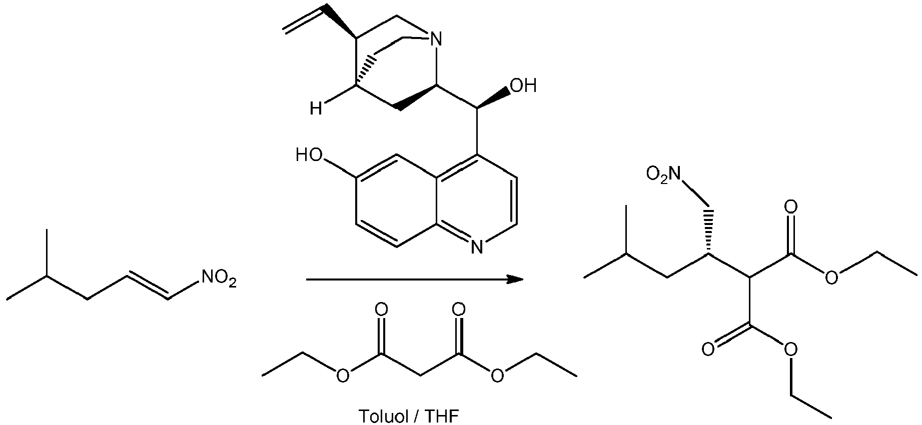

Zu einer Lösung von (E)-4-Methyl-1-nitropent-1-en (19 g, 147 mmol) in 80 mL Toluol und 30 mL THF werden 4,5 g (14,7 mmol) Cupreidin zugegeben. Die Lösung wird auf -20 °C Innentemperatur abgekühlt und 47,14 g Malonsäurediethylester (294,21 mmol) zugegeben. Der Umsatz ist nach 16 Stunden bei -20 °C vollständig. Zur Aufarbeitung wird das Reaktionsgemisch zwei Mal mit je 50 mL 1 N HCl und anschließend mit 50 mL gesättigter, wässriger NH₄Cl-Lösung extrahiert. Nach der Phasentrennung wird die organische Phase mittels azeotroper Destillation getrocknet. Es wird eine 25 %-ige Lösung des Produktes in Toluol mit einem Gesamtgewicht von 147 g erhalten, das entspricht 37 g (3S)-Ethyl-2-acetyl-5-methyl-3-(nitromethyl)hexanoat (86 % Ausbeute).

### Beispiel 3: Darstellung von Kalium-(S)-5-methyl-3-(nitromethyl)hexanoat

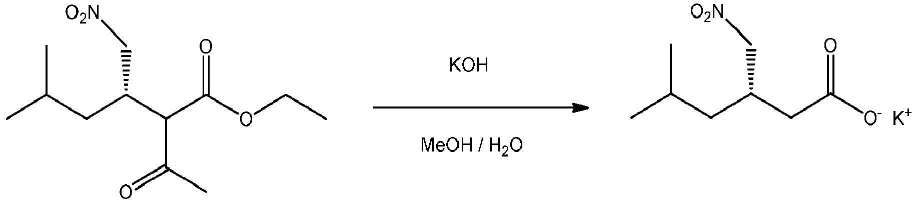

Aus einer Lösung von 785 mg (3,03 mmol) (3S)-Ethyl-2-acetyl-5-methyl-3-(nitromethyl)hexanoat in 3,1 mL Toluol wird das Lösemittel unter Vakuum abgezogen und der Rückstand in einem Gemisch aus 2,15 mL Methanol und 3,95 mL Wasser aufgenommen. Bei 20 °C werden 4,13 g Kaliumhydroxid (80 %-ig, (58,9 mmol)) zum Reaktionsgemisch zugegeben. Nach 120 min bei 50 °C ist kein Edukt mehr vorhanden (Umsatzkontrolle mittels HPLC), der Umsatz somit quantitativ. Gemäß HPLC wird eine Ausbeute von 90 % Kalium-(S)-5-methyl-3-(nitromethyl)hexanoat erhalten. Die Lösung wird allerdings nicht weiter aufgereinigt, sondern direkt dem nächsten Schritt (4.) zugeführt.

¹H NMR (D₂O, 500MHz): δ = 6.00 (d, *J*=8.2 Hz, 2 H), 2.93 - 3.04 (m, 1 H), 2.29 (dd, *J*=13.7, 5.8 Hz, 1 H), 2.11 (dd, *J*=13.7, 8.7 Hz, 1 H), 1.46 - 1.59 (m, 1 H), 1.12 - 1.37 (m, 2 H), 0.74 - 0.86 (m, 6 H) ppm.

¹³C NMR (D₂O, 126MHz): δ = 181.6, 123.5, 41.4, 40.8, 33.5, 25.6, 22.6, 21.9 ppm.

### Beispiel 4: Darstellung von (S)-3-(Aminomethyl)-5-methylhexansäure (Pregabalin)

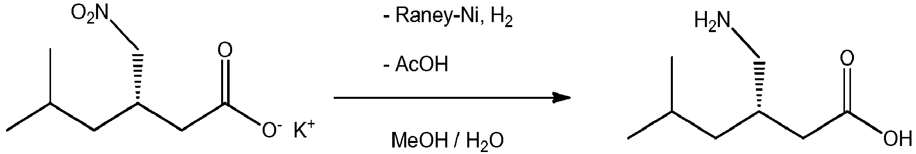

Zu einer Lösung aus 482 mg (2,55 mmol) Kalium-(S)-5-methyl-3-(nitromethyl)hexanoat, 306 mg KOH (5,35 mmol), 1,0 mL Wasser und 2,0 mL Methanol werden 485 mg abdekantierter Raney-Nickel gegeben und unter Wasserstoffatmosphäre (1 bis 5 bar) heftig bei Raumtemperatur gerührt. Nach 12 Stunden wird der Katalysator abfiltriert und ca. 1,0 mL Methanol bei vermindertem Druck abgedampft. Zu dieser Lösung wird nun innerhalb von ca. einer Stunde Eisessig (ca. 382 mg) zugegeben, so dass sich ein pH von 6,9 einstellt. Dabei fällt das Produkt als weißer, kristalliner Feststoff aus. Bei einer Innentemperatur von 2 °C wird eine Stunde nachgerührt und das Produkt anschließend filtriert und getrocknet. Man erhält 345 mg (S)-3-(Aminomethyl)-5-methylhexansäure (Pregabalin), entsprechend einer Ausbeute von 85 %.

ESI-HRMS: berechnet für C₈H₁₇NO₂+H 160,1332, gefunden 160,1321.

¹H NMR (D₂O, 500 MHz): δ = 2.95 (d, *J*=6.3 Hz, 2 H), 2.34 - 2.46 (m, 2 H), 2.14 (spt, *J*=6.7 Hz, 1 H), 1.55 (dquin, *J*=13.5, 6.7 Hz, 1 H), 1.12 - 1.22 (m, 2 H), 0.77 (d, *J*=6.3 Hz, 3 H), 0.80 (d, *J*=6.6 Hz, 3 H) ppm.

¹³C NMR (D₂O, 126MHz): δ = 176.6, 43.3, 40.4, 36.3, 31.1, 24.3, 22.2, 21.7 ppm.

### Beispiel 5: Vergleich unterschiedlicher Katalysatoren bei der Addition von Nucleophilen an (E)-4-Methyl-1-nitropent-1-en

Im zweiten Reaktionsschritt, also der Herstellung von Nitroestern (8), können analog zur Umsetzung mit Cupreidin (Katalysator B) auch die Katalysatoren A, C und D eingesetzt werden. Die jeweiligen Ausbeuten und Werte für den Enatiomerenüberschuss ("ee") bei der Verwendung unterschiedlicher Nucleophile (7) sind in Tabelle 1 wiedergegeben.

**Tabelle 1**

| | Katalysator | Nucleophil | Ausbeute [%] | ee [%] |
|---|---|---|---|---|
| 1 | 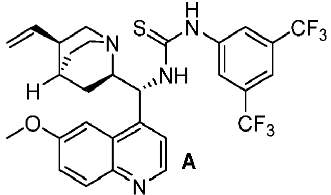 | Malonsäurediethylester | 80 | 57 |
| 2 | 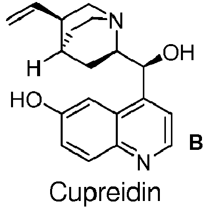 | Malonsäurediethylester | 86 | 94 |
| | | Ethylacetoacetat | 97 | 93* |
| 3 | 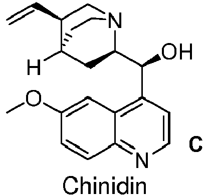 | Malonsäurediethylester | 90 | 45 |
| 4 | 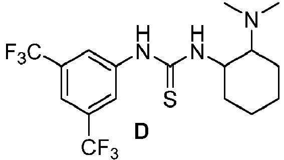 | Malonsäurediethylester | 83 | 80 |

| | | | | |
|---|---|---|---|---|
| * Diastereomerenüberschuss | | | | |

Die Ausbeutebestimmung erfolgte mittels NMR unter Verwendung eines internen Standards.

## Patentansprüche

1. Verfahren zur Herstellung von 1-Nitroalk-1-enen umfassend die Umsetzung eines aliphatischen Aldehyds der Formel (1) mit einem Nitroalkan der Formel (3) in Gegenwart eines Diamins (2) zu einem 1-Nitroalk-1-en der Formel (5) gemäß folgendem Reaktionsschema: 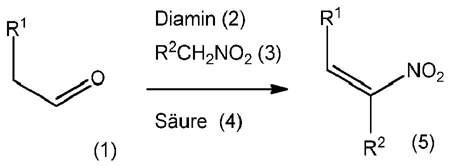 wobei R¹ = C*ₙ*H_{2*n*+1} mit *n* = 0 bis 8 und R² = C*ₘ*H_{2*m*+1} mit *m* = 0 bis 4, **dadurch gekennzeichnet, dass** die Eliminierung zum 1-Nitroalk-1-en durch die Zugabe einer Säure (4) erfolgt.

2. Verfahren gemäß Anspruch 1, wobei der aliphatische Aldeyd der Formel (1) ausgewählt ist aus der Gruppe bestehend aus Ethanal (Acetaldehyd), Propanal (Propionaldehyd), n-Butanal (Butyraldehyd), n-Pentanal (Valeraldehyd), 3-Methylbutanal (Isovaleraldehyd) und n-Hexanal (Capronaldehyd), bevorzugt 3-Methylbutanal (Isovaleraldehyd).

3. Verfahren gemäß Anspruch 1 oder Anspruch 2, wobei das Diamin (2) ausgewählt ist aus der Gruppe bestehend aus N,N-Dimethylethan-1,2-diamin, N,N-Diethylethan-1,2-diamin, N,N-Dipropylethan-1,2-diamin, N,N-Dimethylpropan-1,3-diamin, N,N-Diethylpropan-1,3-diamin, N,N-Dipropylpropan-1,3-diamin, 2-Aminoethylpyrrolidin, 3-Aminopropyl-pyrrolidin, 2-Aminoethylpiperidin und 3-Aminopropylpiperidin, bevorzugt N,N-Dimethylethan-1,2-diamin.

4. Verfahren gemäß einem der vorhergehenden Ansprüche, wobei das Nitroalkan der Formel (3) ausgewählt ist aus der Gruppe bestehend aus Nitromethan, Nitroethan, 1-Nitropropan, 1-Nitrobutan und 1-Nitropentan, bevorzugt Nitromethan.

5. Verfahren gemäß einem der vorhergehenden Ansprüche, wobei die zugegebene Säure (4) ausgewählt ist aus der Gruppe bestehend aus Ameisensäure, Essigsäure, Propionsäure, Benzoesäure, Salzsäure, Schwefelsäure und Salpetersäure, bevorzugt Essigsäure.

6. Verfahren zur Herstellung von Nitroestern der Formel (8) umfassend die Herstellung eines 1-Nitroalk-1-ens gemäß einem der vorhergehenden Ansprüche 1 bis 5 sowie die 1,4 Michael-Addition eines Nucleophils der Formel (7) an das 1-Nitroalk-1-en der Formel (5) in Gegenwart eines Katalysators (6) gemäß folgendem Reaktionsschema: 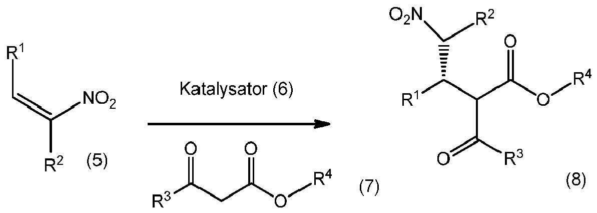 wobei R¹ = C*ₙ*H_{2*n*+1} mit *n* = 0 bis 8; R² = C*ₘ*H_{2*m*+1} mit *m* = 0 bis 4; R³= C*ₚ*H_{2*p*+1} oder OC*ₚ*H_{2*p*+1} mit *p* = 1 bis 3 und R⁴= C*_{q}*H_{2*q*+1} mit *q* = 1 bis 3.

7. Verfahren gemäß Anspruch 6, wobei das Nucleophil der Formel (7) ausgewählt ist aus der Gruppe bestehend aus 3-Oxo-butansäuremethylester; 3-Oxo-butansäureethylester (Ethylacetoacetat), 3-Oxo-butansäurepropylester, 3-Oxo-butansäureisopropylester, Malonsäuredimethylester, Malonsäurediethylester, Malonsäuredipropylester und Malonsäuredüsopropylester, bevorzugt Ethylacetoacetat sowie Malonsäurediethylester.

8. Verfahren gemäß einem der Ansprüche 6 oder 7, wobei der Katalysator (6) ausgewählt ist aus der Gruppe bestehend aus Chinidin und Cupreidin, bevorzugt Cupreidin.

9. Verfahren zur Herstellung von 4-Aminobutansäuren der Formel (12), umfassend die Herstellung von substituierten Nitroestern der Formel (8) nach einem der Ansprüche 6 bis 8 sowie die Umsetzung des substituierten Nitroesters mit einer Base zu einer Nitrosäure der Formel (10) sowie Hydrierung der Nitrosäure gemäß folgendem Reaktionsschema: 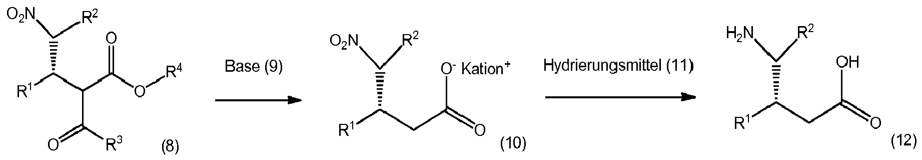 wobei R¹ = C*ₙ*H_{2*n*+1} mit *n* = 0 bis 8; R² = C*ₘ*H_{2*m*+1} mit *m* = 0 bis 4; mit R³= C*ₚ*H_{2*p*+1} oder OC*ₚ*H_{2*p*+1} mit *p* = 1 bis 3 und R⁴= C*_{q}*H_{2*q*+1} mit *q* = 1 bis 3.

10. Verfahren gemäß Anspruch 9, wobei die Base (9) ausgewählt ist aus der Gruppe bestehend aus Kaliumhydroxid, Natriumhydroxid, Magnesiumhydroxid und Calciumhydroxid, bevorzugt Kaliumhydroxid.

11. Verfahren gemäß einem der Ansprüche 9 oder 10, wobei das Hydrierungsmittel (11) ausgewählt ist aus der Gruppe bestehend aus Raney-Nickel, Wasserstoff/Palladiumkatalysator, Wasserstoff/Platinkatalysator oder Wasserstoff /Iridiumkatalysator, bevorzugt Raney-Nickel.

12. Verfahren zur Herstellung von (S)-3-(Aminomethyl)-5-methylhexansäure (Pregabalin) umfassend einen oder mehrere der Verfahrensschritte gemäß einem der Ansprüche 1 bis 11), wobei der Aldehyd der Formel (1) Isovaleraldehyd ist und das Nitroalkan (3) Nitromethan ist.

13. Verfahren zur Herstellung von (S)-3-(Aminomethyl)-5-methylhexansäure (Pregabalin) ausgehend von (E)-4-Methyl-1-nitropent-1-en, wobei das (E)-4-Methyl-1-nitropent-1-en hergestellt wird durch die Umsetzung von Isovaleraldehyd mit Nitromethan in Gegenwart eines Diamins der Formel (2), **dadurch gekennzeichnet, dass** die Eliminierung des (E)-4-Methyl-1-nitropent-1-ens durch die Zugabe einer Säure (4) erfolgt.

14. Verfahren gemäß einem der Ansprüche 13, wobei das Diamin der Formel (2) N,N-Dimethylethan-1,2-diamin ist, die Säure (4) und/oder die zweite Säure Essigsäure ist, die Base (9) Kaliumhydroxid ist und das Hydrierungsmittel Raney-Nickel ist.

15. (3S)-Ethyl-2-acetyl-5-methyl-3-(nitromethyl)hexanoat, Salze der (S)-5-methyl-3-(nitromethyl)hexansäure und deren Verwendung zur Herstellung von (S)-3-(Aminomethyl)-5-methylhexansäure (Pregabalin).
